# EUROPEAN PATENT SPECIFICATION

(11) **EP 2 782 070 B1**
(45) Date of publication and mention of the grant of the patent: **28.06.2017**
(21) Application number: 14160350.6
(22) Date of filing: 17.03.2014
(51) Int. Cl.: G06T 19/00, A61B 8/08, A61B 8/00, G01S 15/89, G01S 7/52, A61B 8/06

(54) **Imaging method and device for the cardiovascular system**
Abbildungsverfahren und -vorrichtung für das kardiovaskuläre System
Procédé et dispositif d'imagerie pour le système cardio-vasculaire

(30) Priority: 19.03.2013 IT GE20130032
(43) Date of publication of application: 24.09.2014
(73) Proprietor: Esaote S.p.A., 16152 Genova (IT)
(72) Inventor: Forzoni, Leonardo, 51100 Pistoia (IT); Costi, Magda, 50032 Borgo san Lorenzo (FI) (IT)
(74) Representative: Karaghiosoff, Giorgio Alessandro

(56) References cited:
- US-A1- 2005 283 075
- US-A1- 2008 270 095
- US-B1- 6 443 894
- US-B2- 6 966 878

## Description

The present invention relates to an imaging method for the cardiovascular system comprising the following steps:
acquiring a volumetric image comprising scan planes adjacent to each other of a periodically moving object;
identifying at least one time interval of at least one periodic movement of said object;
rearranging said scan planes of said volumetric image on the basis of said time interval, by combining with each other the scan planes that are in the same time position with respect to said time interval.

This type of imaging methods are known and used, as they allow images of periodically moving objects to be acquired, by performing evaluations that take the movement itself into consideration and therefore they bring the volumetric images back to a condition with no movement-related distortion.

An example of such method is described in the document US6966878, wherein an acquisition of a volumetric image of a moving object which repeats a cycle of movement over time is periodically performed.

Therefore a time interval of the periodic movement is identified within the volumetric scan, and the volumetric scan is rearranged based on the time interval.

The document describes the method known as Spatio-Temporal Imaging Correlation (STIC), wherein different periodic patterns over time are detected and temporally-spatially correlated events are reconstructed, and therefore the identification of the time interval occurs by correlating the different scan planes.

In similar methods an external signal acquired contemporaneously with the image is used, as for example an electrocardiogram (ECG), that accurately indicates the time frames of the events involved in the periodic movement of the object, for rearranging the scan planes in a process which is known as "triggering".

The known methods are mainly intended for the imaging of the heart (three-dimensional fetal echocardiography the first one, three-dimensional adult echocardiography the second one), and they are able to guarantee a good investigation of the anatomy and of the physiology of the organ regardless of the movement to which it is periodically subjected.

On the contrary when attention is paid to blood vessels (both arterial and venous ones), the known methods are not sufficient for a good evaluation of the possible anomalies.

For example as regards the arteries, particularly the carotid artery, there is the unsatisfied need for a satisfactory three-dimensional morphologic evaluation made by the user about possible plaques or stenoses.

On the contrary as regards veins, such as for example the internal jugular vein, the known methods do not produce satisfactory results for the three-dimensional study of the lumen, of the possible presence of stenoses/strictures/compressions, or of the movement of the valve leaflets.

Veins particularly have periodic movements that depend on a plurality of reasons, among which the heartbeat and the breathing.

This is more clear above all in the study of the lumen of the internal jugular vein both at rest and when it changes based on different pressure manoeuvres such as Valsalva manoeuvre, Müller manoeuvres or deep breathing.

The present invention aims at overcoming said limits of the prior art by a method such as described hereinbefore, wherein said object is composed of at least one blood vessel with periodic movements, at least constriction and dilation, which method provides the following further steps:
generating and displaying a virtual endo-navigation three-dimensional model by segmenting and reconstructing said volumetric image, wherein the point of view is movable along at least one path, for determining plaques, stenoses, structures and/or for analyzing moving structures inside the vessel.

The structures moving in the vessel preferably can be valve leaflets but also elements on the walls of the vessel, bifurcation portions, damaged portions, etcetera.

The fact of providing a three-dimensional endo-navigation model with at least one path on which the point of view is movable is very advantageous for the substantially tubular shape of the blood vessels.

In order to obtain such result a perspective effect is generated, by combining the image of the section plane wherein the point of view lies with the images of the following section planes.

Therefore the user has available an investigation system wherein possible anomalies, particularly constrictions, plaques or stenoses, or the presence of valve leaflets or their evolution in the position over time, are immediately recognizable after reconstructing the image.

The invention is defined by claims 1 and 11, whose preamble is known from US2005/0283075. According to the present invention said identification of at least one time interval of at least one periodic movement of said blood vessel occurs by comparing said scan planes with each other and by contemporaneously acquiring an electrocardiogram signal and by contemporaneously acquiring a breathing signal.

The comparison of the scan planes with each other is performed by methods conventionally used by the STIC using Fourier time analysis, or an autocorrelation of signal intensity. The contemporaneous acquisition of an ECG signal and a breathing signal on the contrary can be used according to the standard methods of the "triggered" or "gated" type acquisitions.

The breathing signal can be acquired in any mode known in the prior art, preferably by measuring the dilation of the trunk in the chest and/or abdomen area when breathing.

The three methods described are used all together.

The choice of the methods used is made on the basis of what is desired to be displayed and of the anatomical district of interest.

The volumetric image obtained is subjected to a plurality of influences such as for example breath, possible movements and heartbeat for the volumetric reconstruction of the internal jugular vein; as regards the carotid artery the image reconstruction can be influenced by the heartbeat and possible movements of the neck, due for example to unintentional movements, deglutition or other ones.

The STIC method for example can be particularly advantageous in the study of the movement of the valve leaflets of the internal jugular vein.

The acquisition of the breathing signal on the contrary is particularly advantageous in the study of the lumen of the internal jugular vein both at rest and subjected to pressure manoeuvres.

It is possible to combine the different methods for rearranging the scan planes through single parallel processing chains, which process independently from each other the volumetric image, the results being finally combined with each other in a single volumetric image by settable weightings. As the reconstruction of the volumetric image is synchronized based on one or more of the following "triggering", ECG, breath, STIC, the user, once the final volume is obtained, can select which triggering has to be used as the main reconstruction trace, by suitably weighing the contribution of each method or not considering the other ones.

According to the present invention, the contributions of comparing said scan planes one with the other and acquiring an electrocardiogram signal and acquiring a breathing signal in rearranging the scan planes are regulated by a weighted sum.

According to a further embodiment said virtual endo-navigation three-dimensional model provides on the reconstructed image one or more virtual lighting points, the shadows being generated correspondingly to the positioning of said one or more lighting points and said one or more lighting points being movable and settable by the user.

The possibility of moving the virtual lighting points is particularly advantageous for studying the presence of plaques or stenoses in the arteries or for studying possible stenoses or the movement of the valve leaflets in the veins.

The modification of the shadows, can efficaciously point out possible constrictions or anomalies in the walls of the vessel.

The lighting point can be set a priori after generating the three-dimensional model but before moving the point of view, or it can be moved during the movement of the point of view.

According to a preferred embodiment the volumetric image is of the ultrasound type, although other types of imaging can be used.

According to a further embodiment said acquisition is made by B-mode and/or Doppler mode.

The Doppler mode allows flows and speeds to be evaluated, which in turn follow periodic patterns.

In the presence of constrictions and anomalies, moreover the speed is subjected to changes that can be easily detected.

According to a further embodiment the point of view of said virtual endo-navigation is automatically movable, said path being determined by the user.

Therefore, once the three-dimensional model is generated and once the image is reconstructed, the user can trace one or more paths in the image, preferably in the blood vessel.

Therefore the point of view automatically continues along the predetermined path.

According to a further embodiment starting from said three-dimensional model one or more section images along one or more predetermined section planes are generated and displayed, and the lumen of the blood vessel all along the extension of the blood vessel comprised in the volumetric image is calculated.

Preferably the section images are displayed besides the endo-navigation image, and the plane upon which the point of view lies is displayed if it is tangent to the section plane of the image.

As an alternative it is possible to set one or more section planes in relation to the point of view, and the section images are generated during the movement of the point of view.

According to an improvement the point of view of said virtual endo-navigation is automatically movable, said path being identified by the center of the lumen of the blood vessel all along the extension of the blood vessel comprised in the volumetric image.

Therefore the lumen of the vessel all along its extension is calculated, and the center of the vessel lumen is calculated. Therefore the path of the point of view is defined by the set of the centers of the vessel lumen in the adjacent scan planes.

Once the path is automatically defined in this manner, the point of view is caused to automatically continue along such path.

Advantageously it is possible to display a graph showing the lumen of the vessel in relation to the axial position in the three-dimensional model, for better evaluating the constrictions.

The acquisition of said volumetric image can be performed by a motorized 2D probe, wherein a transducer array is rotated in a fan-like acquisition.

A problem of this configuration is that the spatial resolution varies along the depth of the acquired volume.

According to an advantageous embodiment the acquisition of said volumetric image is performed by a parallel scanning probe.

This allows the acquisition of the volumetric image to be performed by the insonification and the reception of echoes in a sequential manner along adjacent scan planes.

According to a further embodiment the probe has a flat emitting surface.

The probe performs a parallel scanning of a linear transducer such that it is able to reconstruct a volume represented by a parallelepiped having the width of the array of the probe and the length equal to the linear length of the scanning track. The depth (height) of the parallelepiped is given by the depth settings that are set such to have the segment of interest of the vessel inside the acquired volume.

The present invention further relates to an imaging device according to claim 11. Preferably the means acquiring a volumetric image comprise an ultrasound probe and a reception signal processing unit for generating images along adjacent scan planes.

These and other characteristics and advantages of the present invention will be more clear from the following description of some embodiments shown in the annexed drawings wherein:
Fig. 1 is a general diagram of the method of the present invention;
Fig.2 is one embodiment of the device of the present invention.

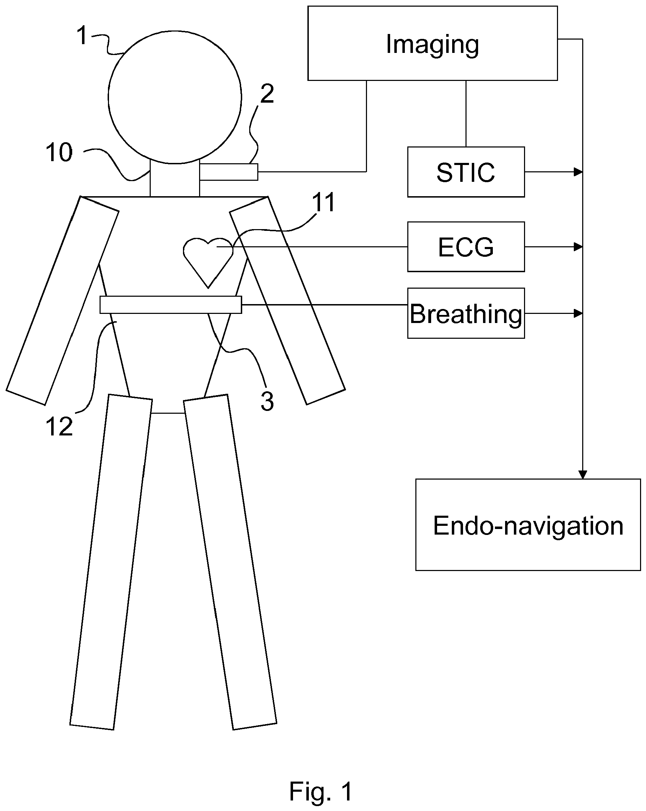

Figure 1 shows a preferred and not limitative embodiment of the imaging method for the cardiovascular system, wherein the volumetric image is acquired from a patient 1, in the anatomical district of the neck 10, particularly for the imaging of the common carotid artery or of the internal jugular vein.

The method provides to acquire a volumetric ultrasound image by means of an ultrasound probe 2 and such volumetric image comprises scan planes adjacent to each other.

The image can be acquired in the B-mode and/or in Doppler mode.

Therefore at least one time interval of at least one periodic movement of said object is identified, particularly dilation and constriction movements, and the scan planes are rearranged based on the time interval, by combining with each other the scan planes that are in the same time position with respect to the time interval.

The identification of the time interval occurs by using, as a combination, a STIC method wherein said scan planes are compared with each other, a ECG and a breathing signal.

The breathing signal is acquired by means 3 measuring the dilation of the trunk 12 in the chest and/or abdomen area during the breathing, which measurement means 3 can be of any type currently known by the person skilled in the art.

Preferably the measurement means 3 comprise one or more straps that can be of the elastic or piezoelectric type, and they can be arranged at the chest and/or abdomen area.

In the case of a single strap, it is preferred to place it at the chest area.

The three methods described are used all together and the choice of the methods used is made on the basis of what is desired to be seen and of the anatomical district of interest.

For example it is possible to consider only the contribution of the ECG method in the case of the imaging of the common carotid artery for a healthy patient.

On the contrary it is advantageous to use the contributions of the ECG method and the STIC method in a patient suffering from neurologic pathology, such to contemporaneously detect the heartbeat and possible unintentional movements, which in turn the more a periodicity characteristic they have the more they can be easily corrected.

The STIC method can easily detect voluntary and/or involuntary repetitive movements as for example swallowing or tremor.

In the case of the internal jugular vein, on the contrary, the influence of the heartbeat and of breathing on the blood flow varies from patient to patient, such as in the same patient from the right side to the left side and also from a body position to a body position, for example supine or sitting position, in the same vein of the same patient.

In this case the characteristics of the flow are detected by the B-mode or by the Color Doppler or Power Doppler mode or by similar technologies for displaying the blood flow and then the combination of "triggering" methods, suitably weighted, is selected, which is the most suitable for reconstructing the volumetric image.

Once the image is reconstructed a virtual endo-navigation three-dimensional model is generated by suitably segmenting and reconstructing the volumetric image.

Therefore a view of the three-dimensional model from a specific point of view is displayed, and such point of view is movable within the three-dimensional model along a path, for determining plaques, stenoses or constrictions.

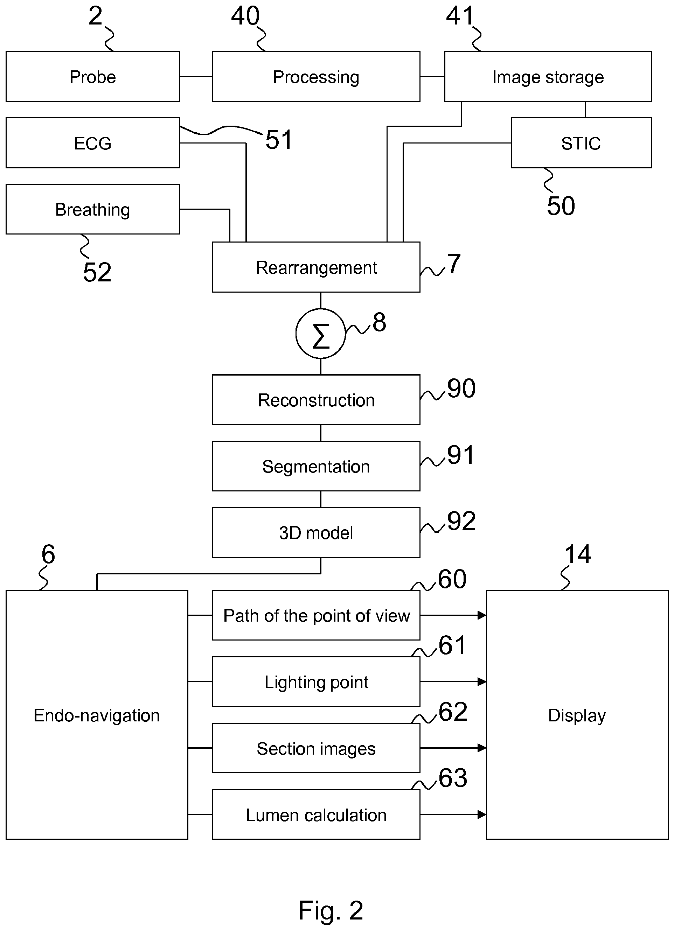

Figure 2 shows the diagram of one embodiment of the device of the present invention, although the boxes can be considered as a functional block diagram of the method.

The device provides an ultrasound probe 2 comprising a linear array of electroacoustic transducers for emitting ultrasound pulses in the body under examination and for receiving echoes generated by the body under examination, means for transmitting to the array electric signals generated by an emission signal generating unit and means for transmitting from the array electric signals to a reception signal processing unit 40 for generating images along adjacent scan planes, and for storing such images in an image storage unit 41.

The probe 2 preferably is provided with means for translating the array along an axis perpendicular to the longitudinal axis of the array from an initial position to a final position and preferably it has a flat emitting surface.

In a preferred embodiment the probe is provided with a pair of rails or tracks and the transducer array is arranged to translate along the rails during acquisition, in a direction perpendicular to its axis.

This parallel linear scanning probe is able to create a parallelepiped volume, and the linear array of transducers acquires perfectly parallel planes characterized by high spatial resolution non geometrically dependent to the different spatial resolution due to depth and scanning angle.

Therefore emissions and receptions are made for a plurality of scan planes during the translation, there being provided means for combining the generated images in a single volumetric image.

Advantageously the movement direction of the array coincides with the longitudinal axis of the blood vessel under examination.

As an alternative it is possible to provide a two-dimensional probe wherein rows of different transducers adjacent to each other are sequentially and individually activated.

With this 3D linear matrix array, the acquisition is performed without the movement of any mechanical part. The array elements are disposed in a matrix, and scan the volume under examination by means of electronic steering. Also in this case, the acquired volume is a parallelepiped volume, with a little possible curvature, due to the non completely linear shape of the array.

As a further alternative a manual acquisition can be performed by a 2D linear probe in combination with a tracking system comprising a transmitter close to the patient and a receiver fixed to the probe.

The transmitter and receiver are preferably electromagnetic as described for example in document EP2706372.

The tracking system enables combining different 3D ultrasound volumes and the nagivation within on the basis of the positioning information detected by means of the electromagnetic field. Two approaches can be used: in a first approch a global 3D reconstruction is performed, based only on the geometric and position information given by the probe position and orientation within the electromagnetic field; in a second approach, in aaddition to the information coming from the tracking system, a data analysis is performed focusing on tissue strutcture recognition, in order to find the best matching betreen the volumes. This second approach can be particularly useful to compensate little tissue compression by the ultrasound probe during scanning.

This method allows to obtain not only parallelepiped volumes but also other shapes.

There are provided means 51 for detecting an ECG signal, means 52 for detecting a breathing signal and a STIC analysis unit 50.

The obtained data are sent from a rearranging unit, which takes the images from the storage unit 41 and performs the rearrangement of the scan planes of the volumetric image on the basis of the time interval defined by the ECG signal detecting means 51, by the breathing signal detecting means 52 and by the STIC analysis unit 50.

The contributions of said methods in rearranging the scan planes of the volumetric image are regulated by a combination unit 8 that carries out a weighted sum of the outputs deriving from the rearranging unit 7.

Preferably the combination unit 8 uses image fusion systems of the feature oriented type, that is built for maximizing the displaying of specific effects or characteristics.

Therefore a three-dimensional model is generated starting from the volumetric image by a reconstructing unit 90, a segmentation unit 91 for recognizing the detected anatomical parts, particularly the walls of the blood vessel, and a 3D model generating unit 92.

Therefore the three-dimensional model is displayed on a display 14 by rendering by means of an endo-navigation system 6, particularly a view of the three-dimensional model from a specific point of view is displayed.

The endo-navigation system 6 comprises means for defining one or more paths of the point of view 60, along which the point of view is movable automatically or under the control of the user.

The path can be established by the user before the real virtual endo-navigation, by means tracing the path in the three-dimensional model.

Moreover there are provided means calculating the lumen of the blood vessel 63, which therefore measure the width of the vessel for all its extension comprised in the volumetric image.

It is possible to provide automatic means signalling anomalies, which signal anomalous constrictions beyond threshold values settable by the user or obtainable from databases of reference values or known clinical cases.

The lumen calculation means 63 further define the centre of the lumen of the blood vessel for each scan plane.

The set of such points for all the scan planes can be used by the means determining the path of the point of view 60 for defining the automatic movement that has to be followed by the point of view.

There are further provided means generating section images 62 from said three-dimensional model.

Such section images are generated along one or more predetermined section planes, preferably two planes on which the longitudinal axis of the blood vessel at least partially lies and which are perpendicular to each other, and a third transverse plane perpendicular to the two first planes.

Preferably the section images generated are displayed on the display 14 besides the endo-navigation image, and the plane on the which the point of view lies is displayed if it is tangent to the plane of the section image.

As an alternative it is possible to set one or more section planes in relation to the point of view, preferably again three planes perpendicular to each other, and the section images are generated during the movement of the point of view.

It is further possible to display on the display 14 a graph showing the lumen of the vessel in relation to the axial position in the three-dimensional model.

The device further provides means generating one or more virtual lighting points 61.

The shadows are generated correspondingly to the positioning of the lighting points which are movable and settable by the user.

The shadows are generated by the known shading and rendering systems.

The lighting point can be set a priori after generating the three-dimensional model but before moving the point of view, or it can be moved by the user during the movement of the point of view.

## Claims

1. Imaging method for the cardiovascular system comprising the following steps:
acquiring a volumetric image comprising scan planes adjacent to each other of a periodically moving object;
identifying at least one time interval of at least one periodic movement of said object;
rearranging said scan planes of said volumetric image on the basis of said time interval, by combining with each other the scan planes that are in the same time position with respect to said at least one interval;
said object being composed of at least one blood vessel with periodic movements, particularly constriction and dilation movements, and the method provides the following further steps:
generating and displaying a virtual endo-navigation three-dimensional model by segmenting and reconstructing said volumetric image, wherein a point of view is movable along at least one path, for determining plaques, stenoses, structures and/or for analyzing moving structures inside the vessel,**characterized in that**
said identification of said at least one time interval of said at least one periodic movement of said blood vessel occurs by using the methods of comparing said scan planes one with the other and of contemporaneously acquiring an electrocardiogram (ECG) signal and of contemporaneously acquiring a breathing signal and
wherein the scan planes rearranged on the basis of said at least one time interval, identified by using said methods, are weighted summed and wherein said comparing the scan planes one with the other is performed by Spatio-Temporal Imaging Correlation (STIC) using Fourier time analysis or autocorrelation of signal intensity.

2. A method according to claim 1 in which single parallel processing chains are provided for processing independently the volumetric image according to the methods and the rearranging of the scan planes, the results of each processing chain being combined with each other in a single volumetric image by settable weightings.

3. A method according to claim 2 in which the weightings are set according to one of the following options:
maintaining in the single volumetric image only the scan planes rearranged on the basis of the time interval identified by acquiring said ECG signal when imaging the common carotid artery of a healthy patient;
using the scan planes rearranged on the basis of the time interval identified by acquiring said ECG signal and by said comparing for contemporaneously detecting heartbeat and unintentional movements;
using the scan planes rearranged on the basis of the time interval identified by acquiring said ECG signal and by acquiring said breathing signal when imaging the internal jugular vein.

4. Method according to claim 1 wherein said virtual endo-navigation three-dimensional model provides on the reconstructed volumetric image one or more virtual lighting points, shadows being generated correspondingly to positioning of said one or more lighting points and said one or more lighting points being movable and settable by a user.

5. Method according to claim 1, wherein said acquisition of said volumetric image is made by B-mode and/or Color Doppler and/or other flow displaying modes.

6. Method according to claim 1, wherein the point of view of said virtual endo-navigation is automatically movable, said path being predetermined by a user.

7. Method according to claim 1, wherein from said three-dimensional model one or more section images along one or more predetermined section planes are generated and displayed, and the lumen of the blood vessel is calculated all along the extension of the blood vessel comprised in the volumetric image.

8. Method according to claim 5, wherein the point of view of said virtual endo-navigation is automatically movable, said path being defined by the centre of the lumen of the blood vessel for each scan plane, wherein the set of such points for all scan planes is used for determining automatically the path of the point of view all along the extension of the blood vessel comprised in the volumetric image.

9. Method according to claim 1, wherein the acquisition of said volumetric image is made by a parallel scanning ultrasound probe.

10. Method according to claim 1, wherein there are provided automatic means for signalling anomalies, which signal anomalous structures beyond threshold values settable by a user or obtainable from databases of reference values or of known clinical cases.

11. Imaging device for the cardiovascular system comprising:
means (2, 40) acquiring a volumetric image comprising scan planes adjacent to each other of a periodically moving object;
means (50, 51, 52) identifying at least one time interval of at least one periodic movement of said object;
means (7) for rearranging said scan planes of said volumetric image based on said time interval, which rearranging means (7) combine with each other the scan planes that are in the same time position with respect to said at least one interval;
said object being composed of at least one blood vessel with periodic movements, particularly constriction and dilation movements, , and the device further comprises:
means (90, 91, 92, 14) for generating and displaying a virtual endo-navigation three-dimensional model by segmenting and reconstructing said volumetric image, wherein a point of view is movable along at least one path, for determining plaques, stenoses, structures and/or for analyzing moving structures inside the vessel, wherein the identifying means comprise electrocardiogram (ECG) signal detecting means (51) for identifying said at least one time interval, and breathing signal detecting means (52) for identifying said at least one time interval; **characterised in that** the identifying means comprise a Spatio-temporal Imaging Correlation (STIC) image analysis unit (50) for identifying said at least one time interval by comparing the scan planes one with the other using Fourier time analysis or autocorrelation of signal intensity ;
and **in that** a combination unit (8) is provided for carrying out a weighted sum of the rearranged scan planes deriving from the rearranging means (7) on the basis of said identified at least one time interval.

## Patentansprüche

1. Bildgebendes Verfahren für das kardiovaskuläre System, umfassend die folgenden Schritte:
Aufnehmen eines volumetrischen Bildes mit aneinander grenzenden Scanebenen eines sich periodisch bewegenden Objekts,
Identifizieren mindestens eines Zeitintervalls mindestens einer periodischen Bewegung des Objekts,
Umordnen der Scanebenen des volumetrischen Bildes auf Basis des Zeitintervalls durch Kombinieren der Scanebenen miteinander, die sich an derselben Zeitposition mit Bezug auf das mindestens eine Intervall befinden,
wobei das Objekt aus mindestens einem Blutgefäß mit periodischen Bewegungen besteht, insbesondere Konstriktions- und Dilations-Bewegungen,
und wobei das Verfahren die folgenden weiteren Schritte vorsieht:
Erzeugen und Anzeigen eines virtuellen dreidimensionalen Endonavigationsmodells durch Segmentieren und Rekonstruieren des volumetrischen Bildes, wobei ein Blickpunkt entlang mindestens einer Bahn bewegbar ist, um Plaques, Stenosen, Strukturen zu bestimmen und/oder um sich bewegende Strukturen innerhalb des Gefäßes zu analysieren, **dadurch gekennzeichnet, dass**
die Identifizierung des mindestens einen Zeitintervalls der mindestens einen periodischen Bewegung des Blutgefäßes durch Verwenden der Verfahren des Vergleichens der Scanebenen miteinander und des zeitgleichen Aufnehmens eines Elektrokardiogramm (EKG)-Signals und des zeitgleichen Aufnehmens eines Atmungssignals stattfindet, und
wobei die auf Basis des mindestens einen durch Verwenden der Verfahren identifizierten Zeitintervalls umgeordneten Scanebenen gewichtet aufsummiert werden, und
wobei das Vergleichen der Scanebenen miteinander durch räumlich-zeitliche Bildkorrelation ("Spatio-Temporal Imaging Correlation", STIC) unter Verwendung von Fourier-Zeitanalyse oder Autokorrelation der Signalintensität ausgeführt wird.

2. Verfahren nach Anspruch 1, bei dem einzelne parallele Verarbeitungsketten zum unabhängigen Verarbeiten des volumetrischen Bildes gemäß den Verfahren und zum Umordnen der Scanebenen vorgesehen werden, wobei die Ergebnisse jeder Verarbeitungskette durch festlegbare Gewichtungen zu einem einzelnen volumetrischen Bild miteinander kombiniert werden.

3. Verfahren nach Anspruch 2, bei dem die Gewichtungen gemäß einer der folgenden Optionen festgelegt werden:
Beibehalten, in dem einzelnen volumetrischen Bild, nur der Scanebenen, die auf Basis des Zeitintervalls umgeordnet werden, das durch Aufnehmen des EKG-Signals identifiziert wird, wenn die Karotis-Arterie eines gesunden Patienten aufgenommen wird,
Verwenden der Scanebenen, die auf Basis des Zeitintervalls umgeordnet werden, das durch Aufnehmens des EKG-Signals und durch das Vergleichen auf zeitgleiches Erfassen von Herzschlag und unbeabsichtigten Bewegungen identifiziert wird,
Verwenden der Scanebenen, die auf Basis des Zeitintervalls umgeordnet werden, das durch Aufnehmens des EKG-Signals und durch Aufnehmen des Atmungssignals identifiziert wird, wenn die innere Jugularvene aufgenommen wird.

4. Verfahren nach Anspruch 1, wobei das virtuelle dreidimensionale Endonavigationsmodell auf dem rekonstruierten volumetrischen Bild einen oder mehrere Beleuchtungspunkte vorsieht, wobei Schatten entsprechend der Positionierung des einen oder der mehreren Beleuchtungspunkte erzeugt werden, und wobei der eine oder die mehreren Beleuchtungspunkte durch einen Benutzer bewegbar und einstellbar sind.

5. Verfahren nach Anspruch 1, wobei das Aufnehmen des volumetrischen Bildes im B- und/oder Farbdoppler-Mode und/oder anderen Durchflussanzeige-Modes erfolgt.

6. Verfahren nach Anspruch 1, wobei der Blickpunkt der virtuellen Endonavigation automatisch bewegbar ist, wobei die Bahn von einem Benutzer vorab festgelegt wird.

7. Verfahren nach Anspruch 1, wobei aus dem dreidimensionalen Modell ein oder mehrere Schnittbilder entlang einer oder mehreren vorbestimmten Schnittebenen erzeugt und angezeigt werden und das Lumen des Blutgefäßes komplett entlang der Ausdehnung des Blutgefäßes in dem volumetrischen Bild berechnet wird.

8. Verfahren nach Anspruch 5, wobei der Blickpunkt der virtuellen Endonavigation automatisch bewegbar ist, wobei die Bahn durch die Mitte des Lumens des Blutgefäßes für jede Scanebene definiert wird, wobei der Satz derartiger Punkte für alle Scanebenen zum automatischen Bestimmen der Bahn des Blickpunkts komplett entlang der Ausdehnung des Blutgefäßes in dem volumetrischen Bild verwendet wird.

9. Verfahren nach Anspruch 1, wobei das Aufnehmen des volumetrischen Bildes durch eine parallel abtastende Ultraschallsonde erfolgt.

10. Verfahren nach Anspruch 1, wobei automatische Mittel zum Signalisieren von Anomalien vorgesehen werden, welche anomale Strukturen jenseits von Schwellenwerten signalisieren, die durch einen Benutzer festlegbar sind oder aus Datenbanken von Referenzwerten oder von bekannten klinischen Fällen erhältlich sind.

11. Bildgebende Vorrichtung für das kardiovaskuläre System, umfassend:
Mittel (2, 40) zum Aufnehmen eines volumetrischen Bildes mit aneinander grenzenden Scanebenen eines sich periodisch bewegenden Objekts,
Mittel (50, 51, 52) zum Identifizieren mindestens eines Zeitintervalls mindestens einer periodischen Bewegung des Objekts,
Mittel (7) zum Umordnen der Scanebenen des volumetrischen Bildes auf Basis des Zeitintervalls, wobei die Umordnungsmittel (7) die Scanebenen miteinander kombinieren, die sich an derselben Zeitposition mit Bezug auf das mindestens eine Intervall befinden,
wobei das Objekt aus mindestens einem Blutgefäß mit periodischen Bewegungen besteht, insbesondere Konstriktions- und Dilations-Bewegungen, und wobei die Einrichtung ferner Folgendes aufweist:
Mittel (90, 91, 92, 14) zum Erzeugen und Anzeigen eines virtuellen dreidimensionalen Endonavigationsmodells durch Segmentieren und Rekonstruieren des volumetrischen Bildes, wobei ein Blickpunkt entlang mindestens einer Bahn bewegbar ist, um Plaques, Stenosen, Strukturen zu bestimmen und/oder um sich bewegende Strukturen innerhalb des Gefäßes zu analysieren, wobei die Mittel zum Identifizieren Mittel (51) zum Erfassen eines Elektrokardiogramm (EKG)-Signals zum Identifizieren des mindestens einen Zeitintervalls und Mittel (52) zum Erfassen des Atmungssignals zum Identifizieren des mindestens einen Zeitintervalls umfassen,
**dadurch gekennzeichnet, dass** die Mittel zum Identifizieren eine Bildanalyseeinheit (50) für die räumlich-zeitliche Bildkorrelation ("Spatio-Temporal Imaging Correlation", STIC) zum Identifizieren des mindestens einen Zeitintervalls durch Vergleichen der Scanebenen miteinander unter Verwendung von Fourier-Zeitanalyse oder Autokorrelation der Signalintensität aufweisen,
und dass eine Kombinationseinheit (8) zum Ausführen einer gewichteten Aufsummierung der umgeordneten Scanebenen vorgesehen ist, die sich aus den Umordnungsmitteln (7) auf Basis des identifizierten, mindestens einen Zeitintervalls ergeben.

## Revendications

1. Procédé d'imagerie pour le système cardio-vasculaire comprenant les étapes suivantes :
saisir une image volumétrique comprenant des plans de balayage situés les uns à côté des autres d'un objet affecté d'un mouvement périodique ;
identifier au moins un intervalle de temps d'au moins un mouvement périodique dudit objet ;
réarranger lesdits plans de balayage de ladite image volumétrique sur la base dudit intervalle de temps, en associant entre eux les plans de balayage qui se trouvent dans la même position de temps par rapport audit au moins un intervalle ;
ledit objet étant composé d'au moins un vaisseau sanguin animé de mouvements périodiques, notamment de mouvements de constriction et de dilatation, et le procédé comporte également les étapes suivantes :
générer et afficher un modèle tridimensionnel d'endonavigation virtuelle par la segmentation et la reconstruction de ladite image volumétrique, dans lequel un point de vue est mobile le long d'au moins une trajectoire pour déterminer des plaques, des sténoses, des structures et/ou pour analyser des structures en mouvement à l'intérieur du vaisseau,
**caractérisé en ce que** ladite identification dudit au moins un intervalle de temps dudit au moins un mouvement périodique dudit vaisseau sanguin est effectuée par des procédés comportant la comparaison desdits plans de balayage l'un avec l'autre, la saisie simultanée d'un signal d'électrocardiogramme (ECG) et la saisie contemporaine d'un signal de respiration et dans lequel une somme pondérée est calculée des plans de balayage réarrangés sur la base dudit au moins un intervalle de temps, identifiés par lesdits procédés, et dans lequel ladite comparaison des plans de balayage l'un avec l'autre est effectuée par une corrélation spatio-temporelle d'images (Spatio-Temporal Imaging Correlation, STIC) au moyen d'une analyse temporelle par transformée de Fourier ou par autocorrélation de l'intensité des signaux.

2. Procédé selon la revendication 1, dans lequel des chaînes de traitement parallèles individuelles sont prévues pour le traitement indépendant de l'image volumétrique selon les procédés et le réarrangement des plans de balayage, les résultats de chaque chaîne de traitement étant combinés dans une seule image volumétrique avec des poids configurables.

3. Procédé selon la revendication 2, dans lequel les poids sont configurés selon l'une des options suivantes :
ne maintenir dans l'image volumétrique individuelle que les plans de balayage réarrangés sur la base de l'intervalle de temps identifié par la saisie dudit signal ECG lors
de la saisie des images de l'artère carotide commune d'un patient sain ;
utiliser les plans de balayage réarrangés sur la base de l'intervalle de temps identifié par la saisie dudit signal ECG et par ladite comparaison pour détecter en même temps le battement cardiaque et les mouvements involontaires ;
utiliser les plans de balayage réarrangés sur la base de l'intervalle de temps identifié par la saisie dudit signal ECG et par la saisie dudit signal de respiration lors de la saisie des images de la veine jugulaire interne.

4. Procédé selon la revendication 1, dans lequel ledit modèle tridimensionnel d'endonavigation virtuelle dispose un ou plusieurs points d'illumination virtuels sur l'image volumétrique reconstruite, des ombres étant générées au niveau du positionnement desdits un ou plusieurs points d'illumination et lesdits un ou plusieurs points d'illumination étant déplaçables et configurables par un utilisateur.

5. Procédé selon la revendication 1, dans lequel ladite saisie de ladite image volumétrique est effectuée par B-mode et/ou Color Doppler et/ou d'autre modes de visualisation des flux.

6. Procédé selon la revendication 1, dans lequel le point de vue de ladite endonavigation virtuelle est déplaçable de façon automatique, ladite trajectoire étant prédéterminée par un utilisateur.

7. Procédé selon la revendication 1 dans lequel, à partir dudit modèle tridimensionnel, une ou plusieurs images de coupe selon un ou plusieurs plans de coupe prédéterminé sont générées et affichées, et le lumen du vaisseau est calculé tout au long du vaisseau sanguin compris dans l'image volumétrique.

8. Procédé selon la revendication 5, dans lequel le point de vue de ladite endonavigation virtuelle est déplaçable de façon automatique, ladite trajectoire étant définie par le centre du lumen du vaisseau sanguin pour chaque plan de coupe, dans lequel l'ensemble de ces points pour tous les plans de coupe est employé pour déterminer de façon automatique la trajectoire du point de vue tout au long du vaisseau sanguin compris dans l'image volumétrique.

9. Procédé selon la revendication 1, dans lequel la saisie de ladite image volumétrique est effectuée par une sonde ultrasonore à balayage parallèle.

10. Procédé selon la revendication 1, dans lequel des moyens de signalisation automatique d'anomalies sont prévues, qui signalent des structures anormales au-dessus de valeurs de seuil qui peuvent être configurées par un utilisateur ou récupérées à partir de bases de données de valeurs de référence ou de cas cliniques connus.

11. Dispositif imagerie pour le système cardio-vasculaire, comprenant :
des moyens (2,40) de saisie d'une image volumétrique comprenant des plans de balayage situés les uns à côté des autres d'un objet affecté d'un mouvement périodique ;
des moyens (50, 51, 52) d'identification d'au moins un intervalle de temps d'au moins un mouvement périodique dudit objet ;
des moyens (7) de réarrangement desdits plans de balayage de ladite image volumétrique sur la base dudit intervalle de temps, lesdits moyens de réarrangement (7) associant entre eux les plans de balayage qui se trouvent dans la même position de temps par rapport audit au moins un intervalle ;
ledit objet étant composé d'au moins un vaisseau sanguin animé de mouvements périodiques, notamment de mouvements de constriction et de dilatation, et le dispositif comprenant par ailleurs : des moyens (90, 91, 92, 14) de génération et d'affichage d'un modèle tridimensionnel d'endonavigation virtuelle par la segmentation et la reconstruction de ladite image volumétrique, dans lequel un point de vue est déplaçable le long d'au moins une trajectoire pour déterminer des plaques, des sténoses, des structures et/ou pour analyser des structures en mouvement à l'intérieur du vaisseau, dans lequel les moyens d'identification comprennent des moyens (51) de détection d'un signal d'électrocardiogramme (ECG) pour identifier ledit au moins un intervalle de temps et des moyens (52) de détection d'un signal de respiration pour identifier le dit au moins un intervalle de temps ; **caractérisé en ce que** les moyens d'identification comprennent une unité (50) d'analyse d'images par corrélation spatio-temporelle d'images (Spatio-Temporal Imaging Correlation, STIC) pour identifier ledit au moins un intervalle de temps en comparant les plans de balayage l'un avec l'autre au moyen d'une analyse temporelle par transformée de Fourier ou par autocorrélation de l'intensité des signaux ;
et **en ce qu'**une unité d'association (8) est prévue pour calculer une somme pondérée des plans de balayage réarrangés dérivant des moyens de réarrangement (7) sur la base dudit au moins un intervalle de temps identifié.
